Europäisches Patentamt

European Patent Office

Office européen des brevets

(19)

(11) Veröffentlichungsnummer: **0 037 889**
**B1**

# EUROPÄISCHE PATENTSCHRIFT

(12)

(45) Veröffentlichungstag der Patentschrift:
02.05.85

(21) Anmeldenummer: 81101249.1

(22) Anmeldetag: 21.02.81

(51) Int. Cl.⁴: **C 07 C 89/02**, C 07 C 91/10

(54) **Verfahren zur Herstellung von 1-Amino-propandiol-(2,3) (III).**

(30) Priorität: **12.04.80 DE 3014129**

(43) Veröffentlichungstag der Anmeldung:
**21.10.81 Patentblatt 81/42**

(45) Bekanntmachung des Hinweises auf die Patenterteilung:
**02.05.85 Patentblatt 85/18**

(84) Benannte Vertragsstaaten:
**BE CH DE FR GB LI NL**

(56) Entgegenhaltungen:
**FR - A - 1 432 428**
**US - A - 3 544 632**

**CHEMICAL ABSTRACTS, Band 79, Nr. 17, 29. Oktober 1973, Seite 406, Nr. 105010n Columbus, Ohio, U.S.A. V.I. PANSEVICH-KOLYADA et al.: "Oxide compounds. Derivatives of methylalkyl(aryl)-substituted glycidols"**

(73) Patentinhaber: **Degussa Aktiengesellschaft, Weissfrauenstrasse 9, D-6000 Frankfurt am Main 1 (DE)**

(72) Erfinder: **Kleemann, Axel, Dr., Greifenhagenstrasse 25, D-6450 Hanau 9 (DE)**
Erfinder: **Nygren, Robert, Dr., Fürstenbergstrasse 8, D-6450 Hanau 9 (DE)**
Erfinder: **Wagner, Rudolf, Dr., Greifenhagenstrasse 9, D-6450 Hanau 9 (DE)**

**Beschreibung**

Die Herstellung von 1-Amino-propandiol-(2,3) durch Addition von Ammoniak an Glycid wurde erstmals von L. Knorr u. E. Knorr (Ber. deutsch. Chem. Ges. 32, 750 (1899) beschrieben. Die Autoren setzten hierbei einen Gewichtsteil Glycid mit 100 Gewichtsteilen 25%igem wässrigem Ammoniak ein und erhielten dann nach destillativer Aufarbeitung 1-Amino-propandiol-(2,3) in einer Ausbeute von 44%, bezogen auf eingesetztes Glycid. Das Gewichtsverhältnis Glycid zu wässrigem Ammoniak (25%ig) = 1:100 bedeutet ein Molverhältnis Glycid zu Ammoniak = 1:109.

Diese Herstellmethode von 1-Amino-propandiol-(2,3) wurde von K. Baum und W.T. Maurice (J. Org. Chem. 27, 2231 (1962) überprüft, wobei dann bei denselben Bedingungen eine Ausbeute von 68% der Theorie erzielt werden konnte. Diese bessere Ausbeute findet ihre Begründung darin, dass die erstgenannten Autoren das Reaktionsprodukt bei 235–250 °C/320 mm Hg destillierten, während die zuletzt genannten Autoren schonender, nämlich bei 80–106 °C/0,1–0,15 mm Hg destillierten und somit keine Verluste durch thermische Zersetzung verursachten.

Wenn auch das zuletzt genannte Verfahren bessere Ausbeuten gegenüber dem Verfahren von Knorr (loc. cit.) bringt, so stellen die im Kreis zu führenden Mengen an wässrigem Ammoniak bei der technischen Durchführung eine erhebliche Belastung dar.

Ausserdem wird durch sie ein sehr grosser Reaktionsraum nötig auf Grund des oben genannten Molverhältnisses von Glycid zu Ammoniak, sowie eine Destillationsanlage zum Konzentrieren der im Kreis zu führenden verdünnten wässrigen Ammoniaklösung.

Wenn es auch nach der US-PS 3 544 632 bekannt ist, Alkanolamine durch Umsetzung von Alkylenoxiden mit Kohlenwasserstoffen und flüssigem Ammoniak in wässrigem Medium umzusetzen, so handelt es sich hier um die Reaktion von monofunktionellen Epoxiden mit Ammoniak, die nicht zur Selbstkondensation neigen.

Zwar wird ein diskontinuierliches Verfahren zur Herstellung der gleichen Stoffe in der FR-PS 1 423 428 beschrieben, bei denen aliphatische Epoxyalkohole eingesetzt werden. Glycid als Anfangsglied wird aber nicht genannt, und bei den in der Patentschrift verwendeten Verbindungen besteht an sich keine Gefahr einer Eigenkondensation. Das Verfahren lässt sich ausserdem nur diskontinuierlich durchführen und ist langwierig.

Zweck des erfindungsgemässen Verfahrens ist daher ein Verfahren zur Herstellung von 1-Amino-propandiol-(2,3) in guten Ausbeuten und auf technisch einfache Weise.

Es wurde nun gefunden, dass man die Umsetzung von Glycid mit Ammoniak in homogener flüssiger Phase mit sehr guten Ausbeuten und ohne besonderen technischen Aufwand durchführen kann, wenn man Glycid und flüssiges Ammoniak unter einem solchen Druck, dass das Ammoniak flüssig bleibt, in Gegenwart einer geringen Menge von organischem Lösungsmittel miteinander umsetzt.

Das Molverhältnis von Glycid zu flüssigem Ammoniak liegt im allgemeinen im Bereich von 1:5 bis 1:20.

Bevorzugt ist ein Molverhältnis von 1:10 bis 20, ganz bevorzugt ein Verhältnis von 1:16 bis 1:17.

Eine Erhöhung des Molverhältnisses über 1:20 hinaus bringt keine erheblichen Ausbeutesteigerungen mehr; unter einem Molverhältnis von 1:5 ist nur noch die technisch gegenüber dem Stand wesentlich vereinfachte Durchführung interessant.

Das Molverhältnis von Glycid zu flüssigem Ammoniak liegt im allgemeinen im Bereich von 1:5 bis 1:20.

Bevorzugt ist das Molverhältnis von 1:10 bis 20.

Der Ausdruck «geringe Menge eines organischen Lösungsmittels» bezieht sich auf die sehr grossen im Stand der Technik (loc. cit.) zu reklyzierenden Wassermengen.

In dem vorliegenden Verfahren werden wesentlich geringere Mengen an organischem Lösungsmittel benötigt. So liegt das Gewichtsverhältnis von Glycid zu dem organischen Lösungsmittel im Bereich von 1:0,2 bis 1:10, bevorzugt ist der Bereich von 1:1.

Der Druckbereich liegt zwischen 5 bis 150 bar, bevorzugt bei 20 bis 90 bar.

Als Reaktionstemperaturen werden 20 °C bis 180 °C, bevorzugt 50–120 °C verwandt.

Temperaturen oberhalb oder unterhalb des genannten Bereiches sind zwar möglich, führen aber unterhalb dieses Bereiches zu unvertretbar langen Reaktionszeiten, oberhalb dieses Bereiches zur verstärkten Nebenproduktbildung.

Der technische Fortschritt des erfindungsgemässen Verfahrens, das sowohl diskontinuierlich wie kontinuierlich durchgeführt werden kann, liegt in der wesentlich – gegenüber dem genannten Stand der Technik – verbesserten Raum-Zeitausbeute und zwar wurde sie gegenüber dem Verfahren von Baum und Maurice (loc. cit.) um den Faktor 50 verbessert.

Ausserdem arbeitet das Verfahren mit stark verringerten Flüssigkeitsmengen, so dass die Rezyklierung vereinfacht wurde.

Es war nicht vorauszusehen, dass dieser technische Fortschritt allein durch die Anwesenheit einer vergleichsweise geringen Menge an organischen Lösungsmitteln möglich wurde.

Das erfindungsgemässe Verfahren wird anhand der folgenden Beispiele näher erläutert:

Versuchsapparatur

Die nachstehenden Beispiele wurden in folgender Versuchsapparatur durchgeführt:

Aus einer mit Glycid bzw. Glycid/organischem Lösungsmittel gefüllten Vorlage und einer Druckflasche mit flüssigem Ammoniak wurden mittels zweier Pumpen mengengeregelt die Reaktanten in den Reaktor gefördert. Dieser bestand aus einem Doppelmantelrohr, wobei der äussere Mantelraum dazu diente, mit Hilfe von organischem Lösungsmittel das Reaktionsgemisch im inneren Rohr auf die gewünschte Temperatur zu bringen und die Reaktionswärme abzuführen. Die Reaktion wurde in flüssiger homogener Phase durchgeführt. Der zur Flüssighaltung der Reaktanten nötige Druck wurde durch ein Druckhalteventil am Ende des Doppelmantelrohres gehalten. Das innere Rohr, also die Reaktionszone, hatte ein Volumen von 4,2 Ltr. Nach Durchlaufen der Reaktionsstrecke wurde das Reaktionsgemisch am Druckregelventil entspannt und in eine Vorlage geleitet. Aus dem so erhaltenen Rohprodukt wurde durch fraktionierte Vakuumdestillation das 1-Aminopropandiol-(2,3) isoliert.

Beispiel 1

In den oben genannten Reaktor wurden bei 85°C und 43 bar pro Stunde 0,5 kg Glycid, 0,5 kg Toluol und 1,7 kg flüssiges Ammoniak eindosiert. (Molverhältnis von Glycid zu Ammoniak = 1:16,8, Gewichtsverhältnis von Glycid zu Toluol = 1:1).

Nach destillativer Aufarbeitung des Reaktionsproduktes erhielt man 0,33 kg Aminopropandiol pro Stunde, entsprechend 53,5% der Theorie, bezogen auf eingesetztes Glycid. Siedepunkt = 94°C (0,2 Torr), Reinheit ≧ 99,5 Gew.-% (Amintitration).

Beispiel 2

In den oben genannten Reaktor wurden bei 85°C und 43 bar pro Stunde 0,6 kg Glycid, 0,6 kg Propanol-(2) und 2,1 kg flüssiges Ammoniak eindosiert. (Molverhältnis von Glycid zu Ammoniak = 1:16, Gewichtsverhältnis von Glycid zu Propanol-(2) = 1:1).

Nach destillativer Aufarbeitung des Reaktionsproduktes erhielt man 0,43 kg Aminopropandiol pro Stunde, entsprechend 58,2% der Theorie, bezogen auf eingesetztes Glycid.

Beispiel 3

In den oben genannten Reaktor wurden bei 85°C und 43 bar pro Stunde 0,7 kg Glycid, 0,7 kg Propanol-(2) und 1,6 kg flüssiges Ammoniak eindosiert. (Molverhältnis von Glycid zu Ammoniak = 1:10, Gewichtsverhältnis von Glycid zu Propanol-(2) = 1:1).

Nach destillativer Aufarbeitung des Reaktionsproduktes erhielt man 0,40 kg Aminopropandiol pro Stunde, entsprechend 54,3% der Theorie, bezogen auf eingesetztes Glycid.

Beispiel 4:

In den oben genannten Reaktor wurden bei 85°C und 43 bar pro Stunde 0,6 kg Glycid, 0,6 kg 1,4-Dioxan und 2,1 kg flüssiges Ammoniak eindosiert. (Molverhältnis von Glycid zu Ammoniak = 1:16, Gewichtsverhältnis von Glycid zu 1,4-Dioxan = 1:1).

Nach destillativer Aufarbeitung des Reaktionsproduktes erhielt man 0,4 kg Aminopropandiol pro Stunde, entsprechend 54,2% der Theorie, bezogen auf eingesetztes Glycid.

Siedepunkte und Reinheit des Produktes in den Beispielen 2–4 entsprechen den Angaben in Beispiel 1.

## Patentansprüche

1. Verfahren zur Herstellung von 1-Aminopropandiol-(2,3) durch Umsetzung von Glycid mit Ammoniak, dadurch gekennzeichnet, dass man Glycid und flüssiges Ammoniak unter einem solchen Druck, dass das Ammoniak in flüssigem Zustand verbleibt, in Gegenwart einer geringen Menge eines mit flüssigem Ammoniak mischbaren organischen Lösungsmittels, und zwar im Gewichtsverhältnis von Glycid zu organischem Lösungsmittel von 1:0,2 bis 1:10, umsetzt.

2. Verfahren nach Anspruch 1, dadurch gekennzeichnet, dass Kohlenwasserstoffe, niedermolekulare primäre oder sekundäre Alkohole oder Glykoläther eingesetzt werden.

3. Verfahren nach Anspruch 1 und 2, dadurch gekennzeichnet, dass als organische Lösungsmittel alkylierte Benzole, wie Toluol, eingesetzt werden.

4. Verfahren nach Anspruch 1 und 2, dadurch gekennzeichnet, dass als Lösungsmittel niedermolekulare aliphatische Alkohole, bevorzugt Methanol, Äthanol, Propanol-1 oder Propanol-2, eingesetzt werden.

5. Verfahren nach Anspruch 1 und 2, dadurch gekennzeichnet, dass als organische Lösungsmittel Äther, wie 1,4-Dioxan oder Tetrahydrofuran, eingesetzt werden.

6. Verfahren nach Anspruch 1–5, dadurch gekennzeichnet, dass man Glycid und flüssiges Ammoniak im Molverhältnis von 1:5 bis 1:20 einsetzt.

7. Verfahren nach Anspruch 1–6, dadurch gekennzeichnet, dass man Glycid und flüssiges Ammoniak im Molverhältnis von 1:10 bis 1:20 miteinander umsetzt.

8. Verfahren nach Anspruch 1–7, dadurch gekennzeichnet, dass man Glycid und das organische Lösungsmittel im Gewichtsverhältnis von 1:1 einsetzt.

9. Verfahren nach Anspruch 1–8, dadurch gekennzeichnet, dass man die Reaktion unter einem Druck von 5 bis 150 bar, bevorzugt von 20 bis 90 bar, durchführt.

## Claims

1. Process for the production of 1-amino-propane-2,3-diol by reacting glycidol with ammonia, characterised in that glycidol and liquid ammonia are reacted in a weight ratio of glycidol to organic solvent of from 1:0,2 to 1:10 under a pressure such that the ammonia remains in liquid state, in the presence of a small quantity of an organic solvent miscible with liquid ammonia.

2. Process according to claim 1, characterised in that hydrocarbons, low-molecular weight primary or secondary alcohols or glycol ethers are used.

3. Process according to claims 1 and 2, characterised in that alkylated benzenes, such as toluene, are used as organic solvent.

4. Process according to claims 1 and 2, characterised in that low-molecular weight aliphatic alcohols, preferably methanol, ethanol, propan-1-ol or propan-2-ol are used as solvent.

5. Process according to claims 1 and 2, characterised in that ethers, such as 1,4-dioxan or tetrahydrofuran are used as organic solvent.

6. Process according to claims 1 to 5, characterised in that glycidol and liquid ammonia are used in a mol ratio of from 1:5 to 1:20.

7. Process according to claims 1 to 6, characterised in that glycidol and liquid ammonia are reacted with each other in a mol ratio of from 1:10 to 1:20.

8. Process according to claims 1 to 7, characterised in that glycidol and the organic solvent are used in a weight ratio of 1:1.

9. Process according to claims 1 to 8, characterised in that the reaction is carried out under a pressure of from 5 to 150 bars, preferably from 20 to 90 bars.

**Revendications**

1. Procédé de préparation de 1-amino-propanediol(2,3) par réaction du glycide avec l'ammoniac, caractérisé en ce qu'on fait réagir le glycide et l'ammoniac liquide sous une pression telle que l'ammoniac reste liquide, en présence d'une faible quantité d'un solvant organique miscible avec l'ammoniac liquide, et ce dans le rapport pondéral glycide/solvant organique de 1:0,2 à 1:10.

2. Procédé selon la revendication 1, caractérisé en ce qu'on utilise des hydrocarbures, des alcools primaires ou secondaires à poids moléculaire peu élevé ou de l'éther glycolique.

3. Procédé selon les revendications 1 et 2, caractérisé en ce qu'on utilise, à titre de solvant organique, des benzènes alkylés, tels que le toluène.

4. Procédé selon les revendications 1 et 2, caractérisé en ce qu'on utilise, à titre de solvant organique, des alcools aliphatiques à poids moléculaire peu élevé, de préférence le méthanol, l'éthanol, le propanol-1 ou le propanol-2.

5. Procédé selon les revendications 1 et 2, caractérisé en ce qu'on utilise, à titre de solvant organique, de l'éther tel que le 1,4-dioxanne ou tétrahydrofuranne.

6. Procédé selon les revendications 1 à 5, caractérisé en ce qu'on utilise le glycide et l'ammoniac liquide dans le rapport molaire de 1:5 à 1:20.

7. Procédé selon les revendications 1 à 6, caractérisé en ce qu'on fait réagir le glycide et l'ammoniac liquide dans le rapport molaire de 1:10 à 1:20.

8. Procédé selon les revendications 1 à 7, caractérisé en ce qu'on utilise le glycide et le solvant organique dans le rapport pondéral 1:1.

9. Procédé selon les revendications 1 à 8, caractérisé en ce qu'on effectue la réaction sous une pression de 5 à 150 bars, de préférence de 20 à 90 bars.